# EUROPEAN PATENT SPECIFICATION

(11) **EP 4 134 024 B1**
(45) Date of publication and mention of the grant of the patent: **13.03.2024**
(21) Application number: 22189570.9
(22) Date of filing: 09.08.2022
(51) Int. Cl.: A61B 17/29, A61B 18/14

(54) **SHAFT RETENTION FEATURES FOR SHAFT-BASED SURGICAL INSTRUMENTS**
WELLENRÜCKHALTEMITTEL FÜR WELLENBASIERTE CHIRURGISCHE INSTRUMENTE
CARACTÉRISTIQUES DE RÉTENTION DE LA TIGE POUR LES INSTRUMENTS CHIRURGICAUX À TIGE

(30) Priority: 10.08.2021 US 202117398547
(43) Date of publication of application: 15.02.2023
(73) Proprietor: Covidien LP, Mansfield, MA 02048 (US)
(72) Inventor: STAMM, Stephen, J, Boulder, 80301 (US); OLSON, Jessica E.C., Boulder, 80301 (US); ESPONDA, Michelle, Boulder, 80301 (US); LACOSTA, Scott N., Boulder, 80301 (US); ROOMI, Amir, Boulder, 80301 (US)
(74) Representative: Maschio & Soames IP Ltd

(56) References cited:
- US-A1- 2016 045 254
- US-A1- 2016 074 101
- US-A1- 2017 245 921

## Description

### BACKGROUND

### Technical Field

The present disclosure relates to surgical instruments and, more particularly, to shaft-based surgical instruments for grasping, treating, and/or dividing tissue incorporating shaft retention features.

### Background of Related Art

Some energy-based surgical instruments, such as energy-based surgical forceps, utilize mechanical clamping action and application of energy, e.g., radio frequency (RF) energy, ultrasonic energy, microwave energy, light energy, thermal energy, etc., to affect hemostasis by heating tissue to coagulate, cauterize, and/or seal tissue. Coagulation may be sufficient to achieve hemostasis on some tissue, e.g., non-vascular tissue, small blood vessels below about two millimeters in diameter, and tissues including small vessels. However, for other tissue, e.g., large blood vessels above about two millimeters in diameter and tissues including larger vessels, coagulation may be insufficient to achieve hemostasis; instead, these tissues may be required to be sealed, a process by which the collagen in the tissue is heated up, denatured, and reformed into a fused mass to permanently close the vessel(s). Once hemostasis is achieved, the treated tissue may be cut (mechanically, electrically, or electro-mechanically) to divide the tissue.

US2017245921, US2016074101 and US 2016045254 disclose surgical instruments which provide background to the invention.

### SUMMARY

The invention is defined in claim 1. Further embodiments are defined in the dependent claims. No surgical methods form part of the invention.

As used herein, the term "distal" refers to the portion that is being described which is further from an operator, e.g., a surgeon or a surgical robot, while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, and/or other variations, up to and including plus or minus 10 percent. Further, any or all of the aspects described herein, to the extent consistent, may be used in conjunction with any or all of the other aspects described herein.

Provided in accordance with aspects of the present disclosure is a surgical instrument including a housing, a shaft, an end effector assembly, and a bobbin. The housing defines a distal aperture and includes an internal partition proximally-spaced from the distal aperture to define an interior cavity therebetween. The internal partition defines a shaft aperture therethrough. The shaft includes a proximal end portion disposed within the housing having at least one protrusion. The shaft extends distally through the shaft aperture of the internal partition, the internal cavity, and the distal aperture of the housing to a distal end portion of the shaft that is distally-spaced from the housing. The end effector assembly is supported at the distal end portion of the shaft. The bobbin includes a body, a proximal rim, and a distal cylinder. The bobbin is disposed about the shaft such that the at least one protrusion is captured within a track defined within the distal cylinder. The bobbin is configured for positioning within the housing such that the body extends through the shaft aperture of the internal partition with the proximal rim disposed on a proximal side of the partition and the distal cylinder disposed within the interior cavity on a distal side of the internal partition, thereby rotatably supporting the proximal end portion of the shaft within the housing.

In an aspect of the present disclosure, the bobbin is formed as a single, monolithic piece of material. In such aspects, the distal cylinder of the bobbin may define at least one access opening therethrough to permit manipulation of the at least one protrusion of the shaft with the bobbin disposed about the at least one protrusion. In aspects, the at least one protrusion is at least one tab and the manipulation includes flaring the at least one tab radially outwardly from the shaft.

In another aspect of the present disclosure, the bobbin is formed from first and second pieces. The first piece may include the body, the proximal rim, and a portion of the distal cylinder, while the second piece includes a remaining portion of the distal cylinder. The second piece may be configured to engage the first piece to capture the at least one protrusion within the track.

In still another aspect of the present disclosure, the first and second pieces include semi-cylinders each defining approximately half of the distal cylinder.

In yet another aspect of the present disclosure, the first and second pieces are configured for snap-fit engagement with one another.

In still yet another aspect of the present disclosure, the first piece includes a proximal wall of the distal cylinder and the second piece includes the remainder of the distal cylinder. In such aspects, the proximal wall of the first piece may include a plurality of fingers extending therefrom configured for receipt within cut-outs defined within a distal wall of the second piece. Alternatively or additionally, a plurality of pins may be provided to rotationally lock the first and second pieces with one another.

In another aspect of the present disclosure, the surgical instrument further includes a drive assembly including a movable handle pivotably coupled to the housing and a drive sleeve extending through the shaft and operably coupled between the movable handle and the end effector assembly. Actuation of the movable handle translates the drive sleeve to thereby actuate the end effector assembly.

In another aspect of the present disclosure, the at least one protrusion includes at least one flared tab cut out from and configured to be flared radially outwardly from the shaft.

### BRIEF DESCRIPTION OF THE DRAWINGS

The above and other aspects and features of the present disclosure will become more apparent in view of the following detailed description when taken in conjunction with the accompanying drawings wherein like reference numerals identify similar or identical elements.
FIG. 1 is a perspective view of a shaft-based surgical instrument provided in accordance with the present disclosure;
FIG. 2 is a side perspective view of a proximal portion of the instrument of FIG. 1 with a portion of the housing removed to illustrate internal features therein;
FIG. 3 is an exploded, perspective view of the instrument of FIG. 1;
FIG. 4 is an enlarged, perspective view of a distal end portion of the instrument of FIG. 1;
FIG. 5A is a shaft-retention bobbin provided in accordance with the present disclosure and configured for use with the instrument of FIG. 1;
FIG. 5B is a perspective view of the bobbin of FIG. 5A shown retaining a proximal portion of the shaft of the instrument of FIG. 1 within a portion of the housing thereof;
FIG. 5C is a perspective view of the bobbin of FIG. 5A shown retaining the proximal portion of the shaft of the instrument of FIG. 1 within the portion of the housing thereof with the bobbin is shown in phantom to illustrate features therein;
FIG. 6A is a perspective view of another shaft-retention bobbin provided in accordance with the present disclosure and configured for use with the instrument of FIG. 1;
FIG. 6B is a perspective view of the bobbin of FIG. 6A, in a disassembled condition;
FIG. 6C is a perspective view of the bobbin of FIG. 6A engaged about the proximal portion of the shaft of the instrument of FIG. 1;
FIG. 6D is a perspective view of the bobbin of FIG. 6A disposed about the proximal portion of the shaft of the instrument of FIG. 1 in the disassembled condition;
FIG. 7A is a perspective view of another shaft-retention bobbin provided in accordance with the present disclosure and configured for use with the instrument of FIG. 1;
FIG. 7B is a perspective view of the bobbin of FIG. 7A, in a disassembled condition;
FIG. 7C is a perspective view of the bobbin of FIG. 7A engaged about the proximal portion of the shaft of the instrument of FIG. 1;
FIG. 8A is a perspective view of another shaft-retention bobbin provided in accordance with the present disclosure and configured for use with the instrument of FIG. 1 with a portion of the bobbin shown in phantom to illustrate features therein;
FIG. 8B is a perspective view of the bobbin of FIG. 8A in a disassembled condition with a portion of the bobbin shown in phantom; and
FIG. 8C is a perspective view of the bobbin of FIG. 8A engaged about the proximal portion of the shaft of the instrument of FIG. 1 with a portion of the bobbin shown in phantom.

### DETAILED DESCRIPTION

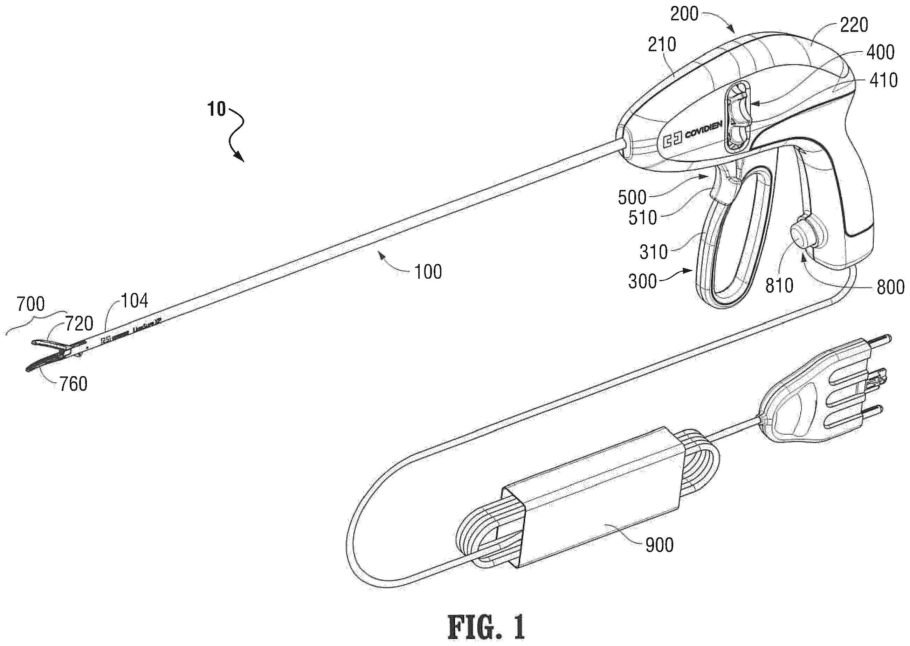

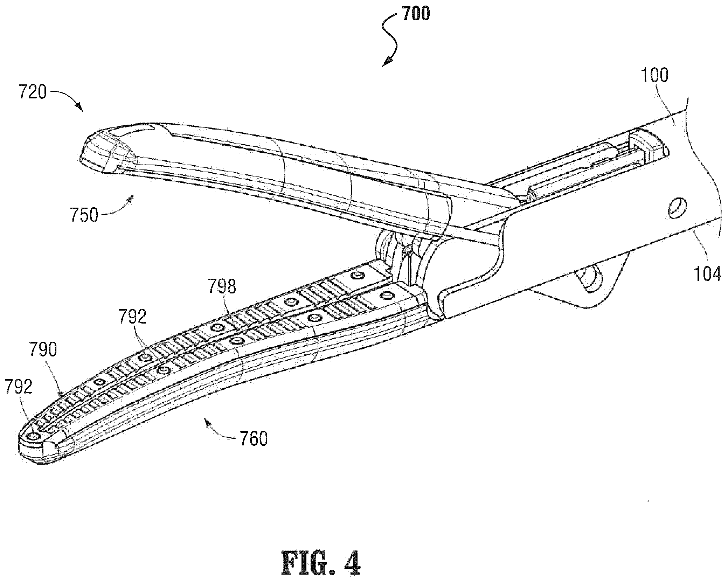

Referring generally to FIGS. 1-4, an energy-based surgical instrument provided in accordance with the present disclosure and configured for grasping, treating, and/or dividing tissue is shown generally identified by reference numeral 10. Instrument 10 includes a shaft 100, a housing 200, a drive assembly 300, a rotation assembly 400, a trigger assembly 500, a knife assembly 600, an end effector assembly 700, an activation assembly 800, and a cable 900.

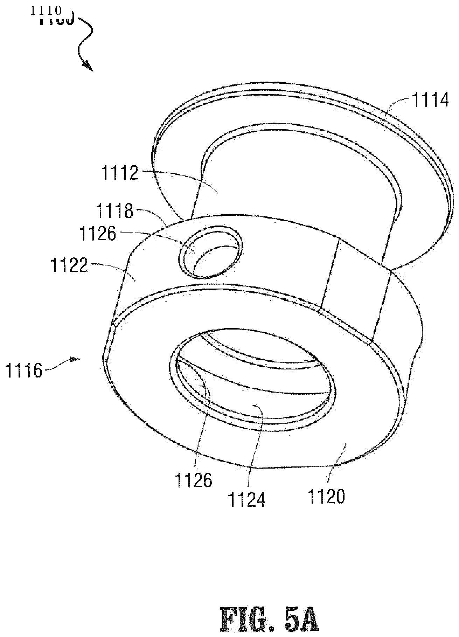

Shaft 100 extends distally from housing 200 and supports end effector assembly 700 at a distal end portion 104 thereof. A bobbin 110 is disposed about a proximal end portion 102 of shaft 100 within housing 200. Bobbin 110 includes a body 112 and a rim 114 disposed at either end of body 112. Bobbin 110 is supported within housing 200, as detailed below, to thereby rotatably support proximal end portion 102 of shaft 100 within housing 200. One or more flared tabs 108 (in aspects, a plurality of flared tabs 108) are cut out from shaft 100 and flared outwardly therefrom. Tabs 108 are positioned distally of bobbin 110 and, due to the radially-outwardly flared configuration thereof, inhibit distal sliding of bobbin 110 along shaft 100 and over tabs 108. In aspects, two diametrically opposed tabs 108 are provided, although greater or fewer tabs 108 and/or other arrangements of tabs 108 are also contemplated. As an alternative to tabs 108, any other suitable protrusion(s) extending radially outwardly from shaft 100 are also contemplated, e.g., one or more bosses extending from shaft 100, a coupler engaged about shaft 100, a flange engaged about or formed with shaft 100, etc. Various different bobbin configurations, engagements thereof about proximal end portion 102 of shaft 100, and/or rotatable supporting thereof within housing 200 are described in detail below with reference to FIGS. 5A-8C.

Housing 200 of instrument 10 includes first and second housing parts 210, 220 secured to one another, e.g., via ultrasonic welding, to operably support and/or enclose various components of instrument 10 within housing 200. More specifically, housing parts 210, 220 may either or both define a distal aperture 244 through which shaft 100 extends distally from housing 200 and a partition 254, e.g., formed from one or more transverse internal walls, defining a shaft aperture 256 that is proximally-spaced from distal aperture 244 of housing 200 such that an internal cavity 258 is defined between distal aperture 244 at the distal end of housing 200 and partition 254. Bobbin 110 is support within housing 200 about partition 254, e.g., with body 112 extending through shaft aperture 256 and rims 114 disposed on either side of partition 254. Shaft 100 is positioned such that tabs 108 (or other suitable protrusion(s)) are disposed within internal cavity 258 between distal aperture 244 at the distal end of housing 200 and partition 254. In this manner, shaft 100 is rotational relative to housing 200 and is constrained but permitted some amount of longitudinal motion due to clearance within internal cavity 258 that allows shaft to move between a proximal position wherein tabs 108 abut the distal rim 114 of proximal collar 110 and a distal position wherein tabs 108 abut the distal internal surface of housing 200. This longitudinal motion, whereby movement of the shaft 100 and end effector assembly 700 relative to the housing 200 is permitted to a relatively small extent, facilitates actuation of end effector assembly 700 while inhibiting over-drive of the drive assembly 300.

Drive assembly 300 includes, among other components, a movable handle 310 that is operably coupled to a drive sleeve 360 within housing 200. Drive sleeve 360 extends from housing 200 through shaft 100 to operably couple with end effector assembly 700 such that pivoting of movable handle 310 relative to housing 200 between an un-actuated position, wherein movable handle 310 is farther spaced-apart from the fixed handle portion of housing 200, and an actuated position, wherein movable handle 310 is more closely approximated relative to the fixed handle portion of housing 200, pivots movable jaw member 720 of end effector assembly 700 relative to fixed jaw member 760 of end effector assembly 700 from a spaced-apart position to an approximated position. Movable handle 310 is further movable towards the fixed handle portion of housing 200 to an activated position to activate activation button 810 of activation assembly 800, although a separate hand switch disposed on housing 200 is also contemplated.

Rotation assembly 400 includes a rotation wheel 410 and a continuous rotation assembly 420. Rotation wheel 410 is engaged about proximal end portion 102 of shaft 100 within housing 200 and extends outwardly through windows defined within either side of housing 200 to enable manual manipulation of rotation wheel 410. More specifically, rotation wheel 410 is continuously rotatable in either direction to thereby rotate shaft 100, and end effector assembly 700 supported by shaft 100, relative to housing 200. Continuous rotation assembly 420 maintains electrical connection of jaw members 720, 760 of end effector assembly 700 with the lead wires from cable 900 and/or activation assembly 800 regardless of the rotational orientation of rotation wheel 410 and shaft 100 relative to housing 200.

Trigger assembly 500 includes a trigger 510 and a plurality of linking structures 520 that operably connect trigger 510 to knife assembly 600 such that actuation of trigger 510 relative to housing 200 from an un-actuated position to an actuated position advances knife assembly 600 to thereby translate knife 610 of knife assembly 600 between jaw members 720, 760 to cut tissue grasped therebetween.

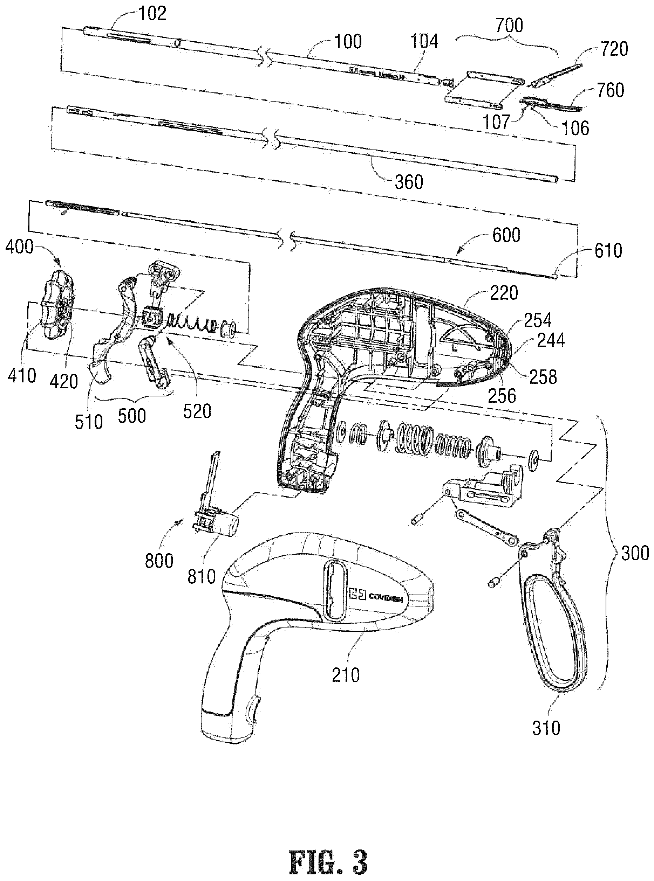

End effector assembly 700 is supported at distal end portion 104 of shaft 100 and includes a movable jaw member 720 and fixed jaw member 760 each including an opposed electrically-conductive plate 750, 790. A pivot pin 106 (FIG. 3) pivotably couples jaw members 720, 760 with one another, while a cam pin 107 (FIG. 3) operably couples drive sleeve 360 to jaw members 720, 760. Thus, translation of drive sleeve 360 through shaft 100 in response to actuation of movable handle 310 relative to housing 200 translates cam pin 107 through cam slots of jaw members 720, 760 to thereby pivot jaw member 720 relative to jaw member 760 and shaft 100 about pivot pin 106 between a spaced-apart position, wherein electrically-conductive plates 750, 790 are farther apart from one another, and an approximated position, wherein electrically-conductive plates 750, 790 are in closer approximation to one another to grasp tissue therebetween.

One or both electrically-conductive plate 750, 790 may include a series of stop members 792 configured to maintain a gap between electrically-conductive plates 750, 790 and inhibit electrical shorting therebetween, e.g., by being formed from an insulative material or by being electrically-isolated from one or both of the electrically-conductive plates 750, 790. Electrically-conductive plates 750, 790 further cooperate to define a knife channel 798 extending longitudinally therethrough in the approximated position of jaw members 720, 760 to facilitate and guide reciprocation of knife 610 of knife assembly 600 (FIG. 3) through jaw members 720, 760 to cut tissue grasped therebetween.

Lead wires (not explicitly shown) are adapted to connect electrically-conductive plates 750, 790 to a source of electrosurgical energy, e.g., an electrosurgical generator (not shown), via cable 900 and continuous rotation assembly 420 of rotation assembly 400 such that, upon activation, electrically-conductive plates 750, 790 are energized to different potentials to enable the conduction of energy therebetween and through the grasped tissue to treat, e.g., seal, the grasped tissue.

Further aspects and features of instrument 10, and more detail regarding the above-noted aspects and features of instrument 10 can be found in U.S. Patent Application Nos. 17/122,062; 17/122,113; and 17/122,144, all filed on December 15, 2020,.

Referring to FIGS. 5A-5C, a bobbin 1110 configured for use in facilitating rotationally support of proximal end portion 102 of shaft 100 within housing 200 is shown. Bobbin 1110 includes a body 1112, a proximal rim 1114 disposed at a proximal end of body 1112, and a distal cylinder 1116 disposed at a distal end of body 1112. Body 1112 is configured to receive proximal end portion 102 of shaft 100 therethrough. Distal cylinder 1116 includes a proximal ring wall 1118, a distal ring wall 1120, and a casing 1122 that cooperate to define an internal annular track 1124 on an interior surface of casing 1122. At least one aperture 1126 extends radially inwardly through casing 1122 to provide access to track 1124 from an exterior of bobbin 1110. Bobbin 1110 may be formed as a single, monolithic piece of material.

Bobbin 1110 is configured for positioning about shaft 100 with flared tabs 108 (or other suitable protrusion(s)) of shaft 100 disposed within distal cylinder 1116. Bobbin 1110, more specifically, is moved into this position prior to the outward flaring of tabs 108. With bobbin 1110 positioned in this manner, a suitable tool(s) (not shown) may be inserted through the one or more apertures 1126 and utilized to flare the one or more tabs 108 radially outwardly from shaft 100 into track 1124 of distal cylinder 1116 of bobbin 1110, thereby longitudinally retaining bobbin 1110 in position about shaft 100.

Bobbin 1110, engaged about shaft 100 as detailed above, is configured for positioning within housing 200 with body 1112 extending through shaft aperture 256, proximal rim 1114 disposed on a proximal side of partition 254 and distal cylinder 1116 disposed on a distal side of partition 254 within internal cavity 258 between distal aperture 244 at the distal end of housing 200 and partition 254. In this manner, proximal end portion 102 of shaft 100 is rotatably supported within housing 200 and longitudinally constrained thereby while still permitting some amount of longitudinal motion due to distal cylinder 1116 defining a length slightly less than a length of internal cavity 258 (that is, a distance between the distal end of housing 200 and partition 254).

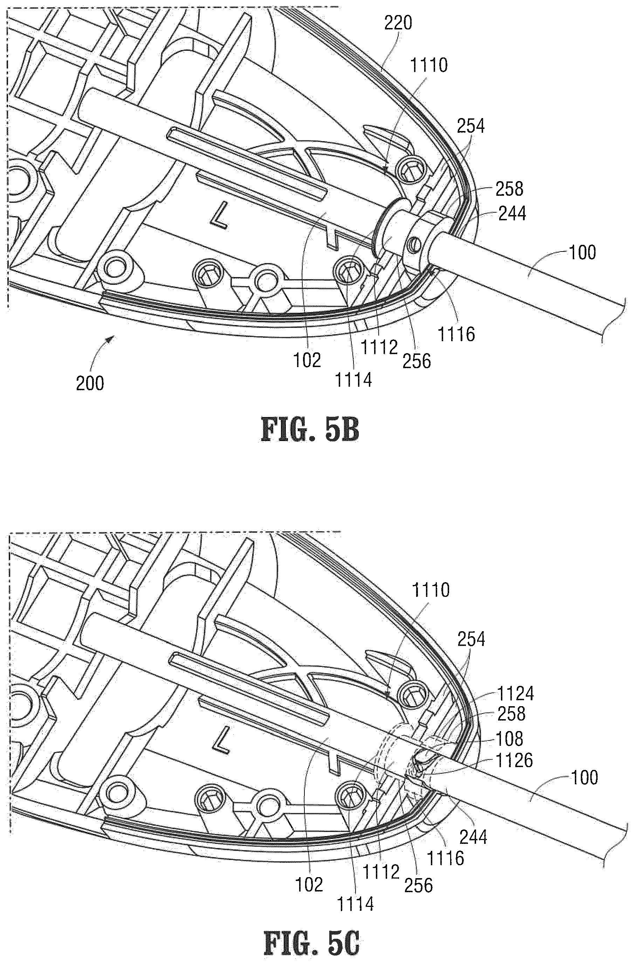

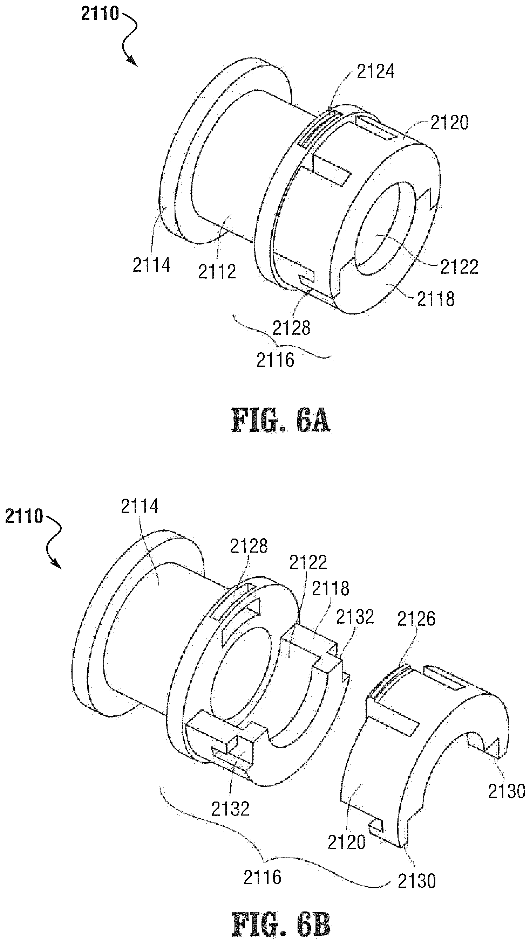

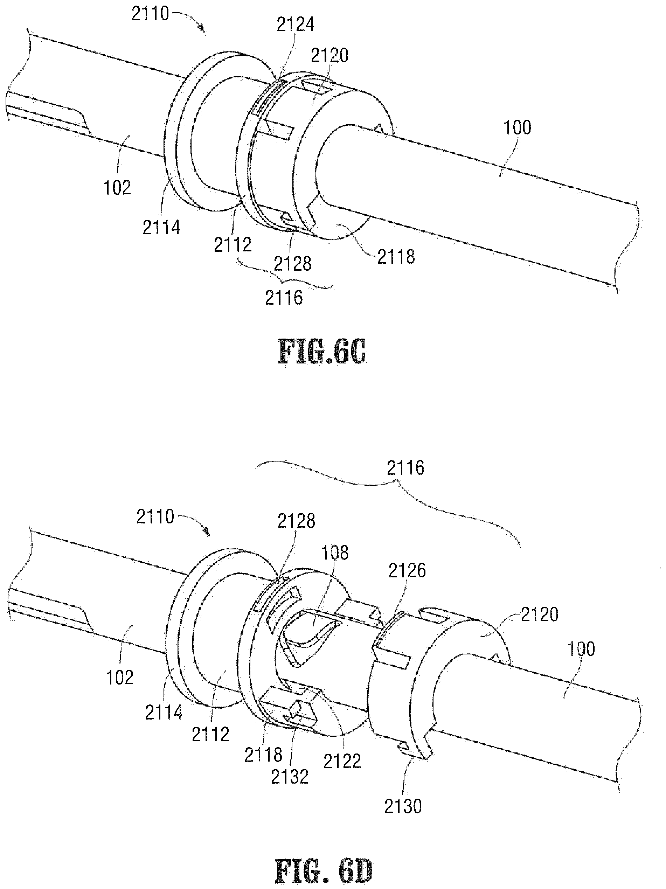

With reference to FIGS. 6A-6D, another bobbin 2110 configured for use in facilitating rotationally support of proximal end portion 102 of shaft 100 within housing 200 is shown (see FIGS. 5B and 5C). Bobbin 2110 is similar to bobbin 1110 (FIGS. 5A-5C) and may include any of the features thereof; thus, only difference between bobbin 2110 and bobbin 1110 (FIGS. 5A-5C) are described in detail below while similarities are summarily described or omitted entirely.

Bobbin 2110 includes a body 2112, a proximal rim 2114 disposed at a proximal end of body 2112, and a two-piece distal cylinder 2116 disposed at a distal end of body 2112. Body 2112, proximal rim 2114, and a first piece 2118 of two-piece distal cylinder 2116 may be formed as a single, monolithic piece of material. Second piece 2120 of distal cylinder 2116 is configured to engage first piece 2118 to form distal cylinder 2116. More specifically, first and second pieces 2118, 2120 may be substantially semi-cylindrical (wherein each defines half a cylinder plus or minus 10%) and configured such that, when engaged with one another, an internal annular track 2122 is defined therein. Other configurations are also contemplated, e.g., wherein one of the pieces 2118, 2120 defines a substantially greater portion of a cylinder than the other.

First and second pieces 2118, 2120 of distal cylinder 2116 may be engagable with one another via a male-female snap-fit connection 2124 wherein one or more male components 2126 is disposed on one or both of first or second pieces 2118, 2120 and one or more female components 2128 are defined on the other or both of first or second pieces 2118, 2120. One or more sets of complementary interfitting alignment structures 2130, 2132 may also be provided on or defined within first and second pieces 2118, 2120 to facilitate alignment thereof during engagement of first and second pieces 2118, 2120 with one another. In aspects, two sets of diametrically-opposed complementary interfitting alignment structures 2130, 2132 are provided.

Bobbin 2110 is configured for positioning about shaft 100 with flared tabs 108 (or other suitable protrusion(s)) of shaft 100 disposed within first piece 2118 of distal cylinder 2116 prior to engagement of second piece 2118b thereon. Bobbin 2110, more specifically, is moved into this position prior to the outward flaring of tabs 108. With bobbin 2110 positioned in this manner, a suitable tool (not shown) may be utilized to flare the one or more tabs 108 radially outwardly from shaft 100. Alternatively, tabs 108 may be flared prior to slidable positioning of bobbin 2110. Once bobbin 2110 is positioned as noted above, second piece 2120 of distal cylinder 2116 may be engaged with first piece 2118 to capture the one or more flared tabs 108 within track 2122 of distal cylinder 2116 of bobbin 2110, thereby rotationally retaining bobbin 2110 about shaft 100. Bobbin 2110 is retained within housing 200 similarly as detailed above with respect to bobbin 1110 (FIGS. 5B and 5C).

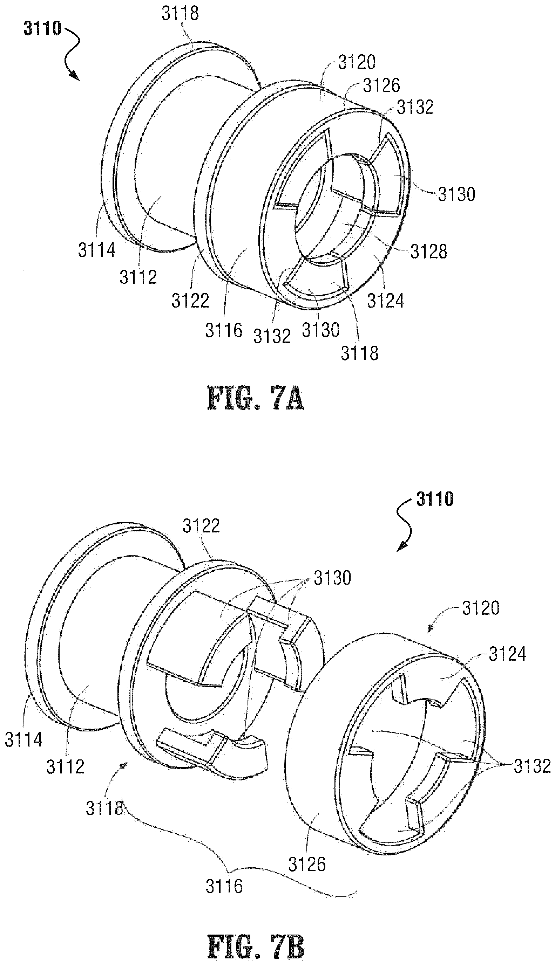

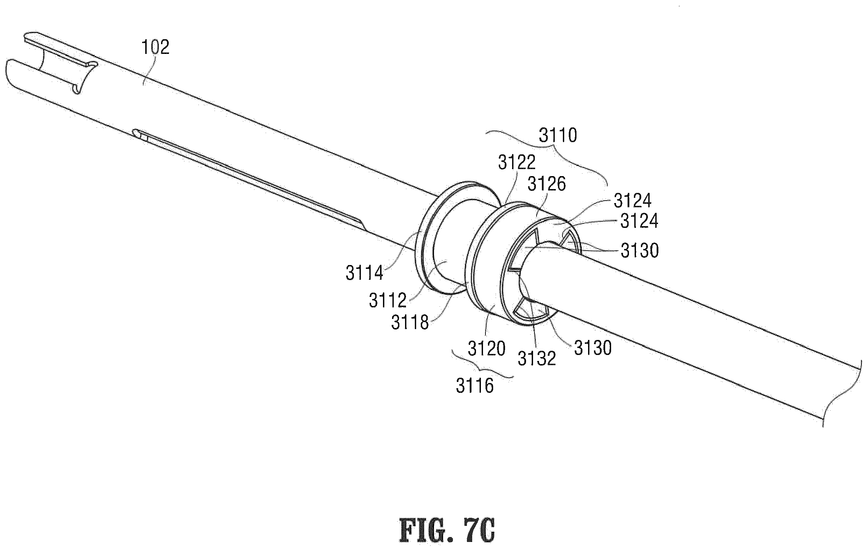

Referring to FIGS. 7A-7C, another bobbin 3110 configured for use in facilitating rotationally support of proximal end portion 102 of shaft 100 within housing 200 is shown (see FIGS. 5B and 5C). Bobbin 3110 is similar to bobbin 2110 (FIGS. 6A-6D) and may include any of the features thereof (including any of the features of bobbin 1110 (FIGS. 5A-5C)); thus, only the different aspects of bobbin 3110 are described in detail below while similarities are summarily described or omitted entirely.

Bobbin 3110 includes a body 3112, a proximal rim 3114 disposed at a proximal end of body 3112, and a two-piece distal cylinder 3116 disposed at a distal end of body 3112. Body 3112, proximal rim 3114, and a first piece 3118 of two-piece distal cylinder 3116 may be formed as a single, monolithic piece of material. Second piece 3120 of distal cylinder 3116 is configured to engage first piece 3118 to form distal cylinder 3116.

First piece 3118 of two-piece distal cylinder 3116 defines the proximal ring wall 3122 of distal cylinder 3116 and includes a plurality of radially-arranged lock fingers 3130 extending distally from proximal ring wall 3122. Second piece 3120 of two-piece distal cylinder 3116 defines the distal ring wall 3124 of distal cylinder 3116 and casing 3126 of distal cylinder 3116 and includes a plurality of radially-arranged lock cut-outs 3132 defined within the distal ring wall 3124.

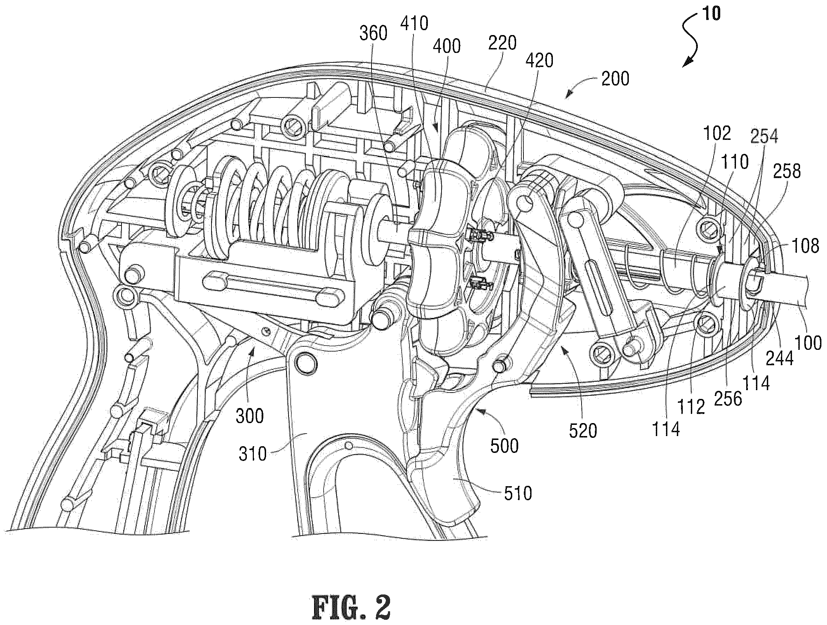

Bobbin 3110 is configured for positioning about shaft 100 with flared tabs 108 (or other suitable protrusion(s)) of shaft 100 disposed within lock fingers 3130 of first piece 3118 of distal cylinder 3116 prior to engagement of second piece 3120 thereon. Bobbin 3110 may be moved into this position prior to the outward flaring of tabs 108 or afterwards. With bobbin 3110 positioned in this manner, second piece 3120 of distal cylinder 3116 is slid proximally over shaft 100 until lock fingers 3130 of first piece 3118 are received through lock cut-outs 3132 of second piece 3120. Once this position is achieved, second piece 3120 is rotated relative to first piece 3118 such that lock fingers 3130 of first piece 3118 and lock cut-outs 3132 of second piece 3120 are at least partially misaligned, thereby locking first and second pieces 3118, 3120 in engagement with one another and capturing the one or more flared tabs 108 (FIG. 2) of shaft 100 within a track 3128 of distal cylinder 3116 of bobbin 3110 formed via the engagement of the first and second pieces 3118, 3120. Thus, bobbin 3110 is rotatably retained about shaft 100. Bobbin 3110 is retained within housing 200 similarly as detailed above with respect to bobbin 1110 (FIGS. 5B and 5C).

As an alternative to the above-detailed rotation-to-lock configuration, first and second pieces 3118, 3120 may also be configured to engage one another via snap-fitting of lock fingers 3130 within lock cut-outs 3132, friction fitting of lock fingers 3130 within lock cut-outs 3132, adhesion, and/or other suitable mechanical, chemical, or other securement technique. In other aspects, no lock feature is provided; rather, with lock fingers 3130 of first piece 3118 received within lock cut-outs 3132 of second piece 3120, thereby rotationally retaining first and second pieces 3118, 3120 relative to one another, the subsequent positioning of distal cylinder 3116 of bobbin 3110 within internal cavity 258 of housing 200 between distal aperture 244 at the distal end of housing 200 and partition 254 of housing 200 retains first and second pieces 3118, 3120 in sufficient approximation with one another due to the presence of partition 254 and the distal end of housing 200 on either side thereof (see FIGS. 5B and 5C).

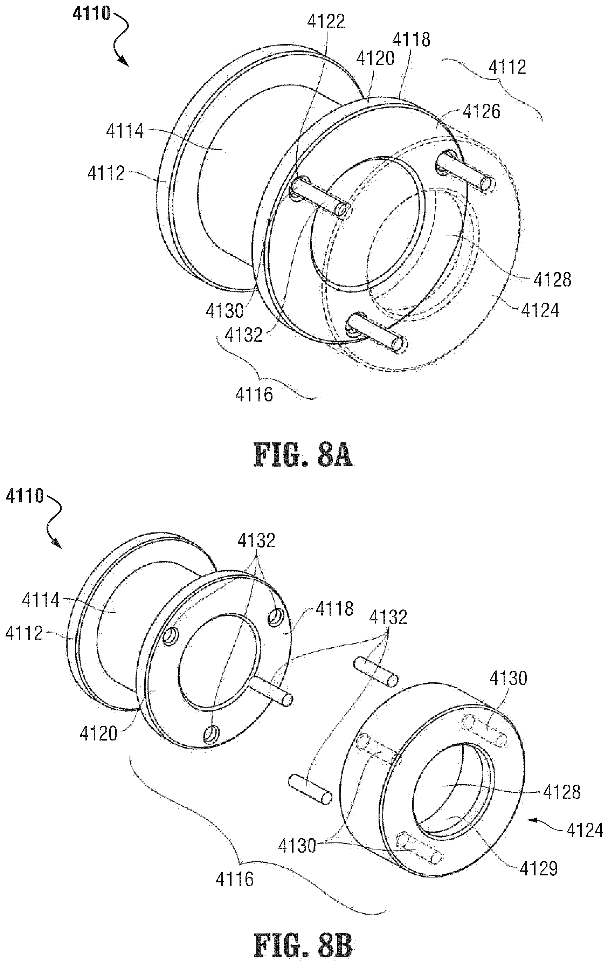

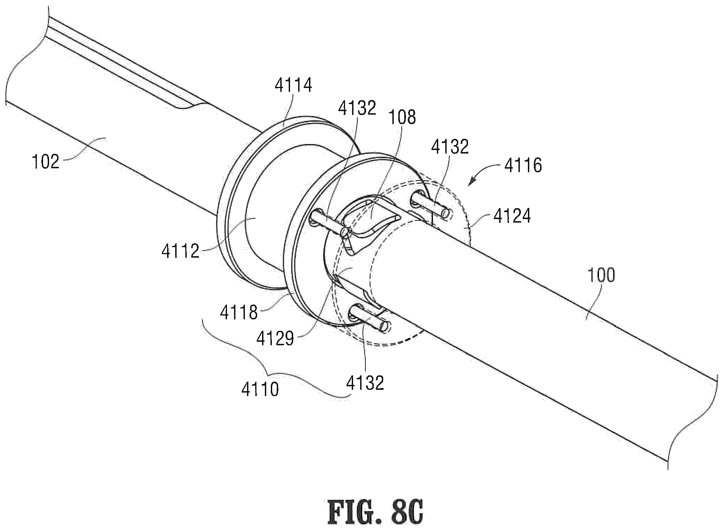

FIGS. 8A-8C, another bobbin 4110 configured for use in facilitating rotationally support of proximal end portion 102 of shaft 100 within housing 200 is shown. Bobbin 4110 is similar to bobbin 3110 (FIGS. 7A-7C) and may include any of the features thereof (including any of the features of bobbin 1110 (FIGS. 5A-5C) or 2110 (FIGS. 6A-6D)); thus, only the different aspects of bobbin 4110 are described in detail below while similarities are summarily described or omitted entirely.

Bobbin 4110 includes a body 4112, a proximal rim 4114 disposed at a proximal end of body 4112, and a two-piece distal cylinder 4116 disposed at a distal end of body 4112 and including a first piece 4118 defining a proximal ring wall 4120 of distal cylinder 4116. A plurality of distally-facing recesses 4122 are defined within a distally-facing surface of proximal ring wall 4120. Second piece 4124 of two-piece distal cylinder 4116 defines a cylinder 4126 itself. Cylinder 4126 defines a lumen 4128 therethrough and includes a plurality of proximally-facing recesses 4130 defined within a proximal face thereof.

Bobbin 4110 is configured for positioning about shaft 100 with flared tabs 108 (or other suitable protrusion(s)) of shaft 100 disposed proximally adjacent proximal ring wall 4120 of first piece 4118 of distal cylinder 4116 prior to engagement of second piece 4124 therewith. Bobbin 4110 may be moved into this position prior to the outward flaring of tabs 108 or afterwards. With bobbin 4110 positioned in this manner, second piece 4124 of distal cylinder 4116 is slid proximally over shaft 100. A plurality of set pins 4132 are configured for receipt within recesses 4122 and 4130 such that, upon further proximal sliding of second piece 4124 of distal cylinder 4116 into approximation with first piece 4118, set pins 4132 rotationally retain first and second pieces 4118, 4124 relative to one another with tabs 108 captured within a track 4129 defined therein. The subsequent positioning of distal cylinder 4116 of bobbin 4110 within internal cavity 258 between distal aperture 244 at the distal end of housing 200 and partition 254 may retain first and second pieces 4118, 4124 in sufficient approximation with one another due to the presence of partition 254 and the distal end of housing 200 on either side thereof (see FIGS. 2 and 5B and 5C). Alternatively or additionally, set pins 4132 may be frictionally retained, retained via adhesive, or retained in any other suitable manner to thereby engage first and second pieces 4118, 4124 with one another.

While several configurations of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular configurations. Those skilled in the art will envision other modifications within the scope and of the claims appended hereto.

## Claims

1. A surgical instrument (10) , comprising:
a housing (200) defining a distal aperture (244) and including an internal partition (254) proximally-spaced from the distal aperture to define an interior cavity (258) therebetween, the internal partition defining a shaft aperture (256) therethrough;
a shaft (100) including a proximal end portion disposed within the housing and having at least one protrusion (108) , the shaft extending distally through the shaft aperture of the internal partition, the internal cavity, and the distal aperture of the housing to a distal end portion of the shaft that is distally-spaced from the housing;
an end effector assembly (700) supported at the distal end portion of the shaft; and
a bobbin (1110; 2110; 3110; 4110) including a body (1112; 2112; 3112; 4112), a proximal rim (1114; 2114; 3114; 4114), and a distal cylinder (1116; 2116; 3116; 4116), the bobbin disposed about the shaft such that the at least one protrusion is rotationally captured within a track (1124; 2122; 3128; 4129)
defined within the distal cylinder, the bobbin configured for positioning within the housing such that the body extends through the shaft aperture of the internal partition with the proximal rim disposed on a proximal side of the partition and the distal cylinder disposed within the interior cavity on a distal side of the internal partition, thereby rotatably supporting the proximal end portion of the shaft within the housing.

2. The surgical instrument according to claim 1, wherein the bobbin is formed as a single, monolithic piece of material.

3. The surgical instrument according to claim 2, wherein the distal cylinder of the bobbin defines at least one access opening therethrough to permit manipulation of the at least one protrusion of the shaft with the bobbin disposed about at least one flared tab.

4. The surgical instrument according to claim 3, wherein the at least one protrusion is at least one tab (108), and wherein the manipulation includes flaring the at least one tab radially outwardly from the shaft.

5. The surgical instrument according to claim 1, wherein the bobbin is formed from first and second pieces, the first piece including the body, the proximal rim (114), and a portion of the distal cylinder, and the second piece including a remaining portion of the distal cylinder, wherein the second piece is configured to engage the first piece to capture the at least one protrusion within the track.

6. The surgical instrument according to claim 5, wherein the first and second pieces include semi-cylinders each defining approximately half of the distal cylinder.

7. The surgical instrument according to claim 5, wherein the first and second pieces are configured for snap-fit engagement with one another.

8. The surgical instrument according to claim 5, wherein the first piece includes a proximal wall of the distal cylinder and wherein the second piece includes the remainder of the distal cylinder.

9. The surgical instrument according to claim 8, wherein the proximal wall of the first piece includes a plurality of fingers extending therefrom configured for receipt within cut-outs defined within a distal wall of the second piece.

10. The surgical instrument according to claim 5, further comprising a plurality of pins rotationally locking the first and second pieces with one another.

11. The surgical instrument according to any preceding claim, further comprising a drive assembly including a movable handle pivotably coupled to the housing and a drive sleeve extending through the shaft and operably coupled between the movable handle and the end effector assembly, wherein actuation of the movable handle translates the drive sleeve to thereby actuate the end effector assembly.

12. The surgical instrument according to any one of claims 5 to 11, wherein the at least one protrusion includes at least one flared tab cut out from and configured to be flared radially outwardly from the shaft.

## Patentansprüche

1. Chirurgisches Instrument (10), umfassend:
ein Gehäuse (200), das eine distale Öffnung (244) definiert und eine innere Trennwand (254) aufweist, die von der distalen Öffnung proximal beabstandet ist, um dazwischen einen inneren Hohlraum (258) zu definieren, wobei die innere Trennwand eine dort hindurchgehende Schaftöffnung (256) definiert,
einen Schaft (100), der einen proximalen Endabschnitt aufweist, der in dem Gehäuse angeordnet ist und mindestens einen Vorsprung (108) hat, wobei sich der Schaft distal durch die Schaftöffnung der inneren Trennwand, den inneren Hohlraum und die distale Öffnung des Gehäuses zu einem distalen Endabschnitt des Schafts erstreckt, der distal von dem Gehäuse beabstandet ist,
eine Endeffektorbaugruppe (700), die an dem distalen Endabschnitt des Schafts gestützt ist, und
eine Spule (1110; 2110; 3110; 4110), die einen Körper (1112; 2112; 3112; 4112), einen proximalen Rand (1114; 2114; 3114; 4114) und einen distalen Zylinder (1116; 2116; 3116; 4116) aufweist, wobei die Spule so um den Schaft angeordnet ist, dass der mindestens eine Vorsprung in einer in dem distalen Zylinder definierten Spur (1124; 2122; 3128; 4129) drehungsmäßig eingefangen ist, wobei die Spule zur Positionierung in dem Gehäuse ausgestaltet ist, so dass sich der Körper durch die Schaftöffnung der inneren Trennwand erstreckt, wobei der proximale Rand an einer proximalen Seite der Trennwand angeordnet ist und der distale Zylinder in dem inneren Hohlraum an einer distalen Seite der inneren Trennwand angeordnet ist, wodurch der proximale Endabschnitt des Schafts drehungsmäßig in dem Gehäuse gestützt ist.

2. Chirurgisches Instrument nach Anspruch 1, wobei die Spule als ein einziges monolithisches Materialteil ausgebildet ist.

3. Chirurgisches Instrument nach Anspruch 2, wobei der distale Zylinder der Spule mindestens eine Zugangsöffnung dort hindurch definiert, um ein Manipulieren des mindestens einen Vorsprungs des Schafts zu gestatten, wobei die Spule um mindestens eine ausgestellte Lasche angeordnet ist.

4. Chirurgisches Instrument nach Anspruch 3, wobei der mindestens eine Vorsprung mindestens eine Lasche (108) ist und wobei zu dem Manipulieren gehört, dass die mindestens eine Lasche von dem Schaft radial nach außen ausgestellt wird.

5. Chirurgisches Instrument nach Anspruch 1, wobei die Spule aus einem ersten und einem zweiten Teil ausgebildet ist, wobei der erste Teil den Körper, den proximalen Rand (114) und einen Abschnitt des distalen Zylinders aufweist, und der zweite Teil einen restlichen Abschnitt des distalen Zylinders aufweist, wobei der zweite Teil dazu ausgestaltet ist, den ersten Teil in Eingriff zu nehmen, um den mindestens einen Vorsprung in der Spur einzufangen.

6. Chirurgisches Instrument nach Anspruch 5, wobei der erste und der zweite Teil Halbzylinder aufweisen, die jeweils ungefähr eine Hälfte des distalen Zylinders definieren.

7. Chirurgisches Instrument nach Anspruch 5, wobei der erste und der zweite Teil dazu ausgestaltet sind, ineinander einzuschnappen.

8. Chirurgisches Instrument nach Anspruch 5, wobei der erste Teil eine proximale Wand des distalen Zylinders aufweist und wobei der zweite Teil des Rest des distalen Zylinders aufweist.

9. Chirurgisches Instrument nach Anspruch 8, wobei die proximale Wand des ersten Teils eine Vielzahl von Fingern aufweist, die sich davon zur Aufnahme in Ausschnitten, die in einer distalen Wand des zweiten Teils definiert sind, erstrecken.

10. Chirurgisches Instrument nach Anspruch 5, ferner umfassend eine Vielzahl von Stiften, die den ersten und den zweiten Teil miteinander drehverriegeln.

11. Chirurgisches Instrument nach einem der vorhergehenden Ansprüche, ferner umfassend eine Antriebsbaugruppe, die einen beweglichen Griff, der schwenkbar an das Gehäuse gekoppelt ist, und eine Antriebshülse aufweist, die sich durch den Schaft erstreckt und zwischen dem beweglichen Griff und der Endeffektorbaugruppe wirkgekoppelt ist, wobei die Antriebshülse durch die Betätigung des beweglichen Griffs translatiert wird, um dadurch die Endeffektorbaugruppe zu betätigen.

12. Chirurgisches Instrument nach einem der Ansprüche 5 bis 11, wobei der mindestens eine Vorsprung mindestens eine ausgestellte Lasche aufweist, die aus dem Schaft ausgeschnitten und dazu ausgestaltet ist, davon radial nach außen ausgestellt zu werden.

## Revendications

1. Instrument chirurgical (10), comprenant :
un boîtier (200) définissant une ouverture distale (244) et comprenant une cloison interne (254) espacée proximalement de l'ouverture distale pour définir une cavité intérieure (258) entre ceux-ci, la cloison interne définissant une ouverture d'arbre (256) à travers celle-ci ;
un arbre (100) comprenant une partie d'extrémité proximale disposée à l'intérieur du boîtier et comportant au moins une saillie (108), l'arbre s'étendant distalement à travers l'ouverture d'arbre de la cloison interne, la cavité interne et l'ouverture distale du boîtier jusqu'à une partie d'extrémité distale de l'arbre qui est espacée distalement du boîtier ;
un ensemble effecteur terminal (700) supporté à la partie d'extrémité distale de l'arbre ; et
une bobine (1110 ; 2110 ; 3110 ; 4110) comprenant un corps (1112 ; 2112 ; 3112 ; 4112), un rebord proximal (1114 ; 2114 ; 3114 ; 4114), et un cylindre distal (1116 ; 2116 ; 3116 ; 4116), la bobine étant disposée autour de l'arbre de telle sorte que l'au moins une saillie soit capturée par rotation à l'intérieur d'une piste (1124 ; 2122 ; 3128 ; 4129) définie à l'intérieur du cylindre distal, la bobine étant configurée pour être positionnée à l'intérieur du boîtier de telle sorte que le corps s'étende à travers l'ouverture d'arbre de la cloison interne, le rebord proximal étant disposé sur un côté proximal de la cloison et le cylindre distal étant disposé à l'intérieur de la cavité intérieure sur un côté distal de la cloison interne, de ce fait supportant en rotation la partie d'extrémité proximale de l'arbre à l'intérieur du boîtier.

2. Instrument chirurgical selon la revendication 1, dans lequel la bobine est formée en tant que pièce de matériau monolithique unique.

3. Instrument chirurgical selon la revendication 2, dans lequel le cylindre distal de la bobine définit au moins une ouverture d'accès à travers celui-ci pour permettre la manipulation de l'au moins une saillie de l'arbre avec la bobine disposée autour d'au moins une languette évasée.

4. Instrument chirurgical selon la revendication 3, dans lequel l'au moins une saillie est au moins une languette (108), et dans lequel la manipulation comprend l'évasement de l'au moins une languette radialement vers l'extérieur de l'arbre.

5. Instrument chirurgical selon la revendication 1, dans lequel la bobine est formée de première et seconde pièces, la première pièce comprenant le corps, le rebord proximal (114) et une partie du cylindre distal, et la seconde pièce comprenant une partie restante du cylindre distal, dans lequel la seconde pièce est configurée pour entrer en prise avec la première pièce afin de capturer l'au moins une saillie à l'intérieur de la piste.

6. Instrument chirurgical selon la revendication 5, dans lequel les première et seconde pièces comprennent des demi-cylindres définissant chacun environ la moitié du cylindre distal.

7. Instrument chirurgical selon la revendication 5, dans lequel les première et seconde pièces sont configurées pour une entrée en prise par encliquetage l'une avec l'autre.

8. Instrument chirurgical selon la revendication 5, dans lequel la première pièce comprend une paroi proximale du cylindre distal et dans lequel la seconde pièce comprend le reste du cylindre distal.

9. Instrument chirurgical selon la revendication 8, dans lequel la paroi proximale de la première pièce comprend une pluralité de doigts s'étendant à partir de celle-ci configurés pour être reçus à l'intérieur de découpes définies à l'intérieur d'une paroi distale de la seconde pièce.

10. Instrument chirurgical selon la revendication 5, comprenant en outre une pluralité de broches verrouillant en rotation les première et seconde pièces l'une avec l'autre.

11. Instrument chirurgical selon une quelconque revendication précédente, comprenant en outre un ensemble d'entraînement comprenant une poignée mobile accouplée de manière pivotante au boîtier et un manchon d'entraînement s'étendant à travers l'arbre et accouplé de manière opérationnelle entre la poignée mobile et l'ensemble effecteur terminal, dans lequel l'actionnement de la poignée mobile déplace par translation le manchon d'entraînement pour actionner ainsi l'ensemble effecteur terminal.

12. Instrument chirurgical selon l'une quelconque des revendications 5 à 11, dans lequel l'au moins une saillie comprend au moins une languette évasée découpée dans l'arbre et configurée pour être évasée radialement vers l'extérieur à partir de celui-ci.
